(19)

**Europäisches Patentamt**
**European Patent Office**
**Office européen des brevets**

(11) **EP 4 190 442 B1**

(12) **EUROPEAN PATENT SPECIFICATION**

(45) Date of publication and mention
of the grant of the patent:
**08.04.2026 Bulletin 2026/15**

(21) Application number: **22210543.9**

(22) Date of filing: **30.11.2022**

(51) International Patent Classification (IPC):
**B01J 20/22** $^{(2006.01)}$ **B01J 20/24** $^{(2006.01)}$
**B01J 20/30** $^{(2006.01)}$

(52) Cooperative Patent Classification (CPC):
**B01J 20/223; B01J 20/24; B01J 20/3071;**
**B01J 20/3085**

(54) **METHOD FOR PRODUCING OF METAL-ALGINATE CROSSLINKED PARTICLES FOR THE REMOVAL OF RESIDUAL ANTIBIOTICS FROM BIOLOGICAL AND ENVIRONMENTAL MEDIA**

VERFAHREN ZUR HERSTELLUNG VON METALL-ALGINAT-VERNETZTEN PARTIKELN ZUR ENTFERNUNG VON ANTIBIOTIKARESTEN AUS BIOLOGISCHEN UND UMWELTMEDIEN

PROCÉDÉ DE PRODUCTION DE PARTICULES RÉTICULÉES À BASE D'ALGINATE MÉTALLIQUE POUR L'ÉLIMINATION D'ANTIBIOTIQUES RÉSIDUELS DE MILIEUX BIOLOGIQUES ET ENVIRONNEMENTAUX

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **01.12.2021 GB 202117355**

(43) Date of publication of application:
**07.06.2023 Bulletin 2023/23**

(73) Proprietor: **Kingston University Higher Education Corporation**
**Kingston Upon Thames, Surrey KT1 1LQ (GB)**

(72) Inventors:
• **KHODER, Mouhamad**
**Kingston Upon Thames, KT1 1LQ (GB)**
• **KARAM, Ayman**
**86000 Poitiers (FR)**

(74) Representative: **Marks & Clerk LLP**
**15 Fetter Lane**
**London EC4A 1BW (GB)**

(56) References cited:
**CN-B- 108 339 526    US-A1- 2007 205 157**

• **CUERVO LUMBAQUE ELISABETH ET AL: "Degradation of pharmaceuticals in different water matrices by a solar homo/heterogeneous photo-Fenton process over modified alginate spheres", ENVIRONMENTAL SCIENCE AND POLLUTION RESEARCH, SPRINGER BERLIN HEIDELBERG, BERLIN/HEIDELBERG, vol. 26, no. 7, 9 January 2019 (2019-01-09), pages 6532 - 6544, XP036739869, ISSN: 0944-1344, [retrieved on 20190109], DOI: 10.1007/S11356-018-04092-Z**
• **HU CHUHUAN ET AL: "Ions-induced gelation of alginate: Mechanisms and applications", INTERNATIONAL JOURNAL OF BIOLOGICAL MACROMOLECULES, ELSEVIER BV, NL, vol. 177, 20 February 2021 (2021-02-20), pages 578 - 588, XP086537264, ISSN: 0141-8130, [retrieved on 20210220], DOI: 10.1016/ J.IJBIOMAC.2021.02.086**

Remarks:
The file contains technical information submitted after the application was filed and not included in this specification

**Description**

This invention relates to the production of metal-alginate crosslinked particles as inactivating agents for the removal of residual antibiotics from biological and environmental media, such as metal-alginate crosslinked particles in which the metal is an ion of iron (Fe), zinc (Zn), nickel (Ni), copper (Cu), aluminium (Al), or calcium (Ca).

Background art

Antimicrobial resistance increasingly poses a grave threat to public health, with potentially enormous human and economic costs [ref 1]. With virtually no new antibiotics created in the new millennia, identifying novel strategies to preserve and extend the useful life of existing antibiotics has become a priority.

Residual antibiotics reaching gastrointestinal and environmental microbiotas are considered as one of the main reasons behind the emergence and spreading of new antimicrobial resistance. A healthy gastrointestinal microbiota is a stable ecosystem that prevents invading microorganisms from colonising the gastrointestinal tract [ref 2]. However, incomplete absorption of antibiotics and their excretion into the intestinal tract disturb the gut's colonisation resistance leading to a microbial vacuum that is readily filled with resistant bacteria. In addition, a high bacterial density, sub-lethal concentrations of residual antibiotics, and a relatively long colonic transit time create a favourable environment for the development of mutational antimicrobial resistance. Subsequently, antimicrobial resistance genes could be horizontally transferred between the microbiota species, before being disseminated into the environment [ref 3].

Antibiotics residues can also reach environment microbiotas from other sources including pharmaceutical industry and hospitals, farms and municipal wastes. Antibiotics contamination can negatively impact environmental ecosystems, leading to the selection and emergence of new antimicrobial resistance that will soon end up in the food chain. Biological and environmental removal and inactivation of residual antibiotics has been suggested as a promising strategy to protect microbiotas, tackle the antimicrobial resistance cycle, and extend the useful life of existing and future antibiotics [refs 4,5]. In order to achieve successful microbiota protection, efficient, bio- and environmentally compatible inactivating agents (IAs), and appropriate delivery or filtration systems, are required. To date, only a few IAs have been tested for biological inactivation of antibiotics residues, whilst more IAs have been used for environmental application.

Residual antibiotics can be eliminated by degradation, adsorption, or complexation. For example, β-lactamase, activated charcoal, and metal-chitosan based microparticles have been tested for their biological (intra-intestinal) inactivation of residual antibiotics [refs 4-8]. Whilst enzymatic inactivation is specific and permanent, it is however limited to a few classes of antibiotics and its clinical and environmental application is hindered by the fragile nature of enzymes. The adsorption approach, using adsorbents such as activated charcoal, has been investigated for the adsorption of intestinal residues of ciprofloxacin [refs 4,5,9,10]. This approach is also widely used to purify aquatic environments from chemical contaminants, including antibiotics. However, adsorbent inactivation is non-specific, implying the possibility of adsorbing other chemicals, hence limiting the overall antibiotics inactivation capacity [ref 4].

Therefore, the development of new effective, safe and specific IAs is still strongly required in order to advance this promising approach to more applicable clinical and environmental usage.

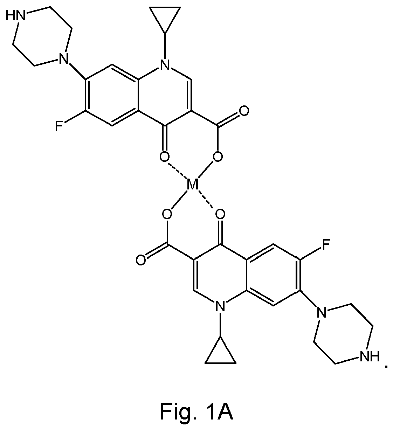

Quinolones such as ciprofloxacin (CIP), levofloxacin (LEV) and norfloxacin (NOR), tetracyclines such as doxycycline (DOXY) and tetracycline (TCN), and beta-lactams such as amoxicillin (AMX), ampicillin (AMP) and cephalexin (CEX) are commonly prescribed for the treatment of a wide range of infections. CIP is used for the treatment of infections caused by gram-negative bacteria. Its absolute bioavailability after oral administration is 70% [refs 11,12]. Furthermore, CIP levels in faeces were reported to reach 251-811 mg/g after parenteral administration [ref 9]. As a result, CIP treatment leads to a profound and long-lasting effect on microbiota composition and the development of mutational resistance by a wide range of the commensal bacteria in the gut [refs 13,14]. The CIP molecule can behave as a unidentate or bidentate ligand towards metals, with respectively one or two molecules of CIP complexed with one metal ion. The more common binding is bidentate (Fig. 1A), which involves the chelation of the metal ion (M) by the carbonyl oxygen at the C-4 position and an oxygen atom on the adjacent carboxyl group [refs 15,16]. Tetracyclines are widely used in human and animal therapy and prophylaxis, exhibiting activity against gram-positive and gram-negative bacteria and parasites. They bind cations and are known to be transported in the blood as metal complexes [ref 28].

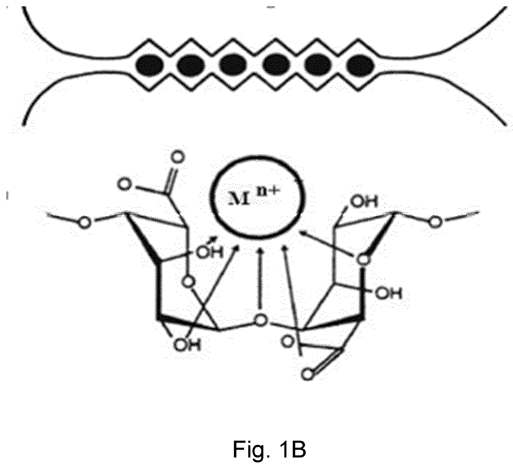

Alginate (ALG) is a naturally-occurring anionic polysaccharide that is safe, biocompatible and available in abundance from renewable sources [refs 17,18]. The carboxylate groups of polyguluronic acid on the ALG backbone can bind cooperatively with specific multivalent cations $M^{n+}$ (e.g. $Ca^{2+}$, $Zn^{2+}$, $Fe^{2+}$, $Fe^{3+}$, etc), leading to chains that crosslink to form an egg-box structure (**Fig. 1B**), and gelation of the ALG solution. While ALG itself does not have specific affinity to quinolones and tetracyclines, metal-ALG crosslinked particles are expected to complex these antibiotics specifically and remove them from aquatic mediums by immobilising them at metal sites within the crosslinked ALG matrix [ref 19].

For example, Zhang et al., Ecotoxological and Environmental Safety, 2019, 169, 392-401 discloses nickel (Ni)-crosslinked alginate particles for inactivating CIP, and reports that the maximum CIP adsorption capacity of the particles is 135.18 mg g$^{-1}$. The Ni-crosslinked alginate particles are prepared by a 'wet' crosslinking method in which a 2% solution of sodium-ALG is dropped through the syringe of an injection pump at 45 mL h$^{-1}$, into a 2% (w/v) Ni$^{2+}$ solution to form gel particles, followed by stirring for 6 hours, filtering, washing and vacuum drying.

Singh et al., Journal of Materials Research and Technology, 2014, 3(3), 195-202 describes iron (Fe)-crosslinked alginate particles and calcium (Ca)-crosslinked alginate particles prepared by emulsion crosslinking methods, and shows that the Fe-alginate particles can reduce bacterial growth and be effective against bacterial contamination in water.

Konwar et al., RSC Advances, 2015, 5, 81573-81582 describes magnetic alginate-Fe$_3$O$_4$ hydrogel fibers, formed by dissolving alginate solution into a dispersion of magnetic Fe$_3$O$_4$ nanoparticles and then squeezing into calcium chloride solution to obtain Ca-crosslinked alginate fibers loaded with iron oxide nanoparticles; or by dipping Ca-crosslinked alginate fibers in a dispersion of magnetic Fe$_3$O$_4$ nanoparticles. CIP adsorption capacities of 423 $\mu$g g$^{-1}$ or 555 $\mu$g g$^{-1}$ are reported for obtained fibers.

The present invention addresses a need for a simpler method to prepare metal-crosslinked alginate particles useful as IAs, in particular iron- or zinc-alginate (Fe-ALG, Zn-ALG) crosslinked particles. The present invention addresses a need for new, more effective IAs for inactivating antibiotics such as ciprofloxacin (CIP), levofloxacin (LEV), norfloxacin (NOR), doxycycline (DOXY), tetracycline (TCN), amoxicillin (AMX), ampicillin (AMP) and cephalexin (CEX).

Further reference may be made to metal-alginate products and their preparation methods disclosed in US 2007/205157 A1, CN108339526 B and Cuervo Lambaque, E et al., Environmental Science and Pollution Research International, ISSN 0944-1344, 26(7), p6532-6544.

Summary of Invention

Accordingly, the present invention provides:

(1) a method for producing metal-alginate crosslinked particles wherein the metal is an ion of iron, zinc, copper, nickel, aluminium, or calcium, comprising:

providing sodium-alginate powder;

providing a solution of a metal salt wherein the metal salt is a salt of iron, zinc, copper, nickel, aluminium, or calcium;

adding the metal salt solution, gradually or portionwise, to the sodium-alginate powder and mixing, to form metal-alginate crosslinked particles in a moist or near-dry state;

drying, or leaving to dry, the formed moist or near-dry metal-alginate crosslinked particles to form dry metal-alginate crosslinked particles;

optionally washing the dry metal-alginate crosslinked particles, followed by filtering and drying;

(2) the method according to (1), wherein the dry metal-alginate crosslinked particles are washed with water and filtered, at least once;

(3) the method according to (1) or (2), wherein the method is for producing metal-alginate crosslinked particles wherein the metal is an ion of iron, zinc, copper, nickel, or aluminium, and the metal salt added is a salt of iron, zinc, copper, nickel, or aluminium;

(4) the method according to any one of (1) to (3), wherein a fluidised bed dryer is used to form the dry metal-alginate crosslinked particles;

(5) the method according to any one of (1) to (4), wherein the method is for producing metal-alginate crosslinked particles wherein the metal is an ion of iron or zinc, and the metal salt added is a salt of iron or zinc;

(6) the method according to any one of (1) to (5), wherein the method is for producing Fe(III)-alginate crosslinked particles, and the solution added is an aqueous iron (III) chloride solution.

The invention also provides:

(7) metal-alginate crosslinked particles prepared by the method of any one of (1) to (5); and
(8) metal-alginate crosslinked particles according to (7), wherein the metal-alginate crosslinked particles are Fe(III)-alginate crosslinked particles prepared by the method of (6).

The invention also provides:

(9) use of metal-alginate crosslinked particles as defined in (7) or (8), as an agent for inactivating antibiotics selected from quinolones, tetracyclines, and beta-lactams;
(10) the use according to (9) for inactivating an antibiotic selected from ciprofloxacin, levofloxacin, norfloxacin, doxycycline, tetracycline, amoxicillin, ampicillin, and cephalexin, preferably ciprofloxacin; and
(11) the use according to (9) or (10), wherein the metal-alginate crosslinked particles in their method of preparation have been washed with water once.

Brief Description of Drawings

Fig.1A shows a CIP bidentate ligand complex with two molecules of CIP complexed to one metal ion M.
Fig.1B shows cooperative binding of carboxylate groups of polyguluronic acid on the ALG backbone with specific multivalent cations $M^{n+}$ leading to chains that crosslink to form an egg-box structure.
Fig. 2A shows vials containing $FeCl_3$ solution.
Figs. 2B-2E show, at intervals, progressive dropwise additions of $FeCl_3$ solution to initially dry Na-ALG powder (Fig. 2B), with mixing, to form moist or near-dry crosslinked alginate particles (Fig. 2E).
Figs. 3A-3F show graphs representing inactivation percentage (%) of antibiotics (100 $\mu$g/mL CIP, LEV, NOR, TCN, DOXY, CEX, AMX, and AMP) in water using 10 mg/mL Dry-Fe-ALG, Dry-Zn-ALG, Dry-Cu-ALG, Dry-Ni-ALG, Dry-Al-ALG, and Dry-Ca-ALG, respectively.
Fig. 4 shows a graph representing inactivation percentage (%) of TCN, DOXY, LEV, and NOR in water (initial antibiotics concentration: 100 $\mu$g/mL) using Wet-Fe-ALG*7w.
Fig. 5A shows a graph representing inactivation percentage (%) of CIP (100 $\mu$g/mL) in water using 10 mg/mL Wet-Fe-ALG*7w, Wet-Zn-ALG*1w, and Wet-Zn-ALG*7w.
Fig. 5B shows a graph representing inactivation percentage (%) of CIP (100 $\mu$g/mL) in water and SIM using 10 mg/mL Wet-Fe-ALG*7w, Wet-Zn-ALG*7w.
Fig. 6A shows a graph representing CIP eliminated ($\mu$g/mL) by 1 mg/mL Wet-Fe (III)-ALG*7w from SIM containing (25, 50,100, 200, 400, 600 mg/L) CIP.
Fig. 6B shows a graph representing an adsorption isotherm of Wet-Fe-ALG*7w / CIP.
Fig. 7A shows a graph representing CIP eliminated ($\mu$g/mL) by 1 mg/mL Dry-Fe-ALG from SIM containing (25, 50,100, 200, 400, 600 mg/L) CIP.
Fig. 7B shows a graph representing an adsorption isotherm of Dry-Fe-ALG / CIP.
Fig. 8 shows the adsorption kinetics of CIP in water using by 1 mg/mL Dry-Fe-ALG.
Fig. 9 shows a bar chart of *E.coli* zones of inhibition using 50 $\mu$L CIP solutions (100 mg/mL) before and after inactivation with Wet-Fe-ALG*7w (1 mg/mL), Dry-Fe-ALG (1 mg/mL), or Wet-Zn-ALG*7w (10 mg/mL).

Detailed Description of Invention

As used herein, "metal-alginate" and "metal-ALG" refer to a complex of alginate (ALG) with metal ions (M), for example "zinc-alginate" (Zn-ALG) refers to an alginate with zinc (Zn) ions. In the "M-ALG" nomenclature used herein, M refers to the relevant metal ion in its stable oxidation state or charge or, if available, in any of its stable oxidation states or charges (preferably its most stable oxidation state or charge), where not specifically indicated. Thus, for example, "Fe-ALG" refers to an alginate of $Fe^{2+}$ and/or $Fe^{3+}$ (preferably $Fe^{3+}$), whereas "Fe(lll)-ALG" refers to an alginate of $Fe^{3+}$ ion; "Zn-ALG" refers to an alginate of $Zn^{2+}$ (zinc ion in its stable oxidation state/charge). In the Examples and Figures herein, however, "Dry-Fe-ALG" and "Wet-Fe-ALG" refer to Fe(III)-ALG prepared by dry and wet crosslinking methods, respectively.

In one aspect, the present invention provides a method for producing metal-alginate crosslinked particles wherein the metal is an ion of iron, zinc, copper, nickel, aluminium, or calcium, comprising:

providing sodium-alginate powder;
providing a solution of a metal salt wherein the metal salt is a salt of iron, zinc, copper, nickel, aluminium, or calcium;
adding the metal salt solution, gradually or portionwise, to the sodium-alginate powder and mixing, to form metal-alginate crosslinked particles in a moist or near-dry state;
drying, or leaving to dry, the formed moist or near-dry metal-alginate crosslinked particles to form dry metal-alginate crosslinked particles;
optionally washing the dry metal-alginate crosslinked particles, followed by filtering and drying.

This method is also herein referred to as a 'dry' method.

The 'dry' method is applied for producing metal-alginate (M-ALG) crosslinked particles, where the metal ion (M) is an ion of a metal selected from iron, zinc, copper, nickel, aluminium, and calcium, i.e., the metal-alginate (M-ALG) is iron-alginate (Fe-ALG), zinc-alginate (Zn-ALG), copper-alginate (Cu-ALG), nickel-alginate (Ni-ALG), aluminium-alginate (Al-ALG), or calcium alginate (Ca-ALG). Preferably, the 'dry' method is applied for producing iron-alginate (Fe-ALG), zinc-alginate (Zn-ALG), copper-alginate (Cu-ALG), nickel-alginate (Ni-ALG), or aluminium-alginate (Al-ALG) crosslinked particles, more preferably for producing Fe-ALG or Zn-ALG crosslinked particles, still more preferably for producing Fe-ALG crosslinked particles, most preferably for producing Fe(III)-ALG crosslinked particles.

According to the dry method, sodium-alginate (Na-ALG) powder is provided, to which is added a solution of a metal salt, the metal salt being chosen as a salt of iron, zinc, copper, nickel, aluminium, or calcium, depending on the metal-alginate crosslinked particles to be produced by the method. Thus, for example, an iron (III) salt solution is added to the Na-ALG powder if the method is applied for producing Fe(III)-ALG crosslinked particles, whereas a zinc salt solution is added if the method is applied for producing Zn-ALG crosslinked particles.

Na-ALG powder is commercially available. Any commercially available Na-ALG powder of any average particle size (fine to coarse) is suitable to produce the metal-ALG crosslinked particles.

Suitable salts, for the metal salt solution to be added to the Na-ALG powder, include any water-soluble salts such as a halide, acetate or sulphate, preferably a chloride or an acetate, more preferably a chloride. The salt for producing Fe-ALG crosslinked particles is preferably $FeCl_3$. The salt for producing Zn-ALG crosslinked particles is preferably $Zn(CH_3COO)_2$.

Suitable solvents, for the solution of the metal salt, include water and aqueous buffers, preferably water. Suitable aqueous buffers are, for example, HEPES buffer and acetate buffer, preferably acetate buffer. The solution is preferably an aqueous solution, in which the solvent preferably consists of water, in particular deionized water.

The concentration of the metal ions in the solution is suitably from dilute to saturated solutions. The higher the concentration, the higher the degree of crosslinking obtained. Most preferably, the metal ion concentration used is close to or at the saturation limit. In the case of $FeCl_3$ as salt, the concentration of iron (III) ions in the solution is preferably from 0.5 M to 2 M, in particular from 1 to 2 M. In the case of $ZnCl_2$ as salt, the concentration of zinc (II) ions in the solution is preferably from 0.5 M to 2 M, in particular from 1 to 2 M.

In this 'dry' method, the metal salt solution is added to the Na-ALG powder and mixed, to form metal-alginate (M-ALG) crosslinked particles (in a moist or near-dry state).

The metal salt solution is added to the Na-ALG powder in an amount to provide a high molar ratio of cations to alginate, in order to achieve a correspondingly high degree of crosslinking. For example, the metal salt solution may be added in an amount to provide a molar ratio of cation:alginate of from 100:1 to 1000:1, for example from 400:1 to 700:1, preferably from 500:1 to 600:1.

The addition of the metal salt solution to the Na-ALG powder should be carried out gradually or portionwise, such as dropwise or as a spray, for example at a rate of 0.1 to 1.0 mL salt solution per minute, typically at a rate of 0.25 mL salt solution per minute, for a duration at least sufficient to obtain the desired degree of crosslinking as dependent on the concentration of the metal salt solution used. Mixing is to be carried out during the addition of the salt solution, preferably continuously during the addition, and may be continued after all of the salt solution has been added.

The addition, with mixing, of the metal salt solution to the Na-ALG powder results in the formation of metal-alginate (M-ALG) crosslinked particles, obtained in a moist or near-dry condition. The obtained M-ALG crosslinked particles are left to dry, or may be dried by subjecting to drying processes known in the art, such as hot air or oven drying, for example at a temperature from 20 to 60°C, for example 25 to 50°C, preferably 35 to 45°C.

In this 'dry' method, for example a fluidised bed dryer can be used to generate the dry M-ALG crosslinked particles. Dry Na-ALG powder particles are lifted from the bottom and suspended in the fluidised bed air stream (fluidised state), whilst the metal salt solution is sprayed via nozzles positioned above and/or below the suspended powder bed to allow the crosslinking and drying to occur at the same time.

The dry or dried M-ALG crosslinked particles may, if desired, be washed, for example with water or buffers, preferably with water, in particular deionized water, and further filtered and dried, to obtain washed, dry M-ALG (e.g. Fe-ALG or Zn-ALG) crosslinked particles.

If desired, the dry or dried M-ALG crosslinked particles may be washed and filtered multiple times, before final drying. The dry or dried M-ALG crosslinked particles are preferably washed, preferably with water, in particular deionized water, and filtered at least once, for example once, twice or three times, preferably once or twice, more preferably once, before final drying.

In another aspect, the present invention provides metal-alginate (M-ALG) crosslinked particles prepared by the above described 'dry' method.

Described as comparison is an alternative method for preparing metal-alginate (M-ALG) crosslinked particles by a so-called 'wet' method. According to this 'wet' method, the following steps are typically carried out:

providing a solution of sodium-alginate;
providing a solution of a metal salt wherein the metal salt is a salt of iron, zinc, copper, nickel, aluminium, or calcium;
adding the sodium-alginate solution to the metal salt solution and mixing, to form wet metal-alginate crosslinked particles;
collecting the wet metal-alginate crosslinked particles;
drying the collected wet metal-alginate crosslinked particles; and
optionally washing the dry metal-alginate crosslinked particles, followed by filtering and drying.

In this 'wet' method, sodium-alginate (Na-ALG) is used in the form of a solution. Suitable solvents for the solution of Na-ALG include water and aqueous buffers, preferably water. Suitable aqueous buffers are, for example, HEPES buffer and acetate buffer, preferably acetate buffer. The solution is preferably an aqueous solution, in which the solvent preferably consists of water, in particular deionized water.

A solution of metal salt is used, the metal salt being chosen as a salt of iron, zinc, copper, nickel, aluminium, or calcium, depending on the metal-alginate crosslinked particles to be produced by the method.

Suitable salts for the metal salt in this 'wet' method include all water-soluble salts such as a halide, acetate or sulphate, preferably a chloride or an acetate, more preferably a chloride. The salt for producing Fe-ALG crosslinked particles is preferably $FeCl_3$. The salt for producing Zn-ALG crosslinked particles is preferably $Zn(CH_3COO)_2$.

Suitable solvents for the solution of the metal salt in this 'wet' method include water and aqueous buffers, preferably water. Suitable aqueous buffers are, for example, HEPES buffer and acetate buffer, preferably acetate buffer. The solution is preferably an aqueous solution, in which the solvent preferably consists of water, in particular deionized water.

In this 'wet' method, the concentration of the metal ions in the solution is suitably from diluted to saturated solution. In the case of $FeCl_3$ as salt, the concentration of iron (III) salts in the solution is preferably from 1 to 10 % w/v. In the case of $Zn(CH_3COO)_2$ as salt, the concentration of zinc (II) ions in the solution is preferably from 1 to 10% w/v.

The Na-ALG solution is added to the metal salt solution and mixed, to form metal-alginate (M-ALG) crosslinked particles (in a wet state).

The Na-ALG solution is added to the metal salt solution in an amount to obtain the desired degree of crosslinking. In this regard, it is preferred to use the metal salt solution in excess to the Na-ALG solution, such that amount such that not all of the metal salt is consumed to crosslink the alginate.

In this 'wet' method, the addition of the Na-ALG solution to the metal salt solution should be carried out gradually or portionwise, such as dropwise or as a spray, typically at a rate of 1 mL of Na-ALG per minute. Any ALG to metal cation molar ratio is suitable to produce metal-ALG crosslinked particle provided the amount and concentration of the metal salt solution used are sufficiently high to obtain the desired degree of crosslinking. Mixing is to be carried out during the addition of the Na-ALG solution, preferably continuously during the addition, and may be continued after all of the Na-ALG solution has been added.

The addition, with mixing, of the Na-ALG solution to the metal salt solution results in the formation of metal-alginate (M-ALG) crosslinked particles, obtained in a wet condition, typically as wet beads in solution such as an aqueous solution. The obtained M-ALG crosslinked particles are collected by filtration and dried by freeze drying or by subjecting them to drying processes known in the art, such as hot air or oven drying, for example at a temperature from 20 to 60°C, for example 25 to 50°C, preferably 35 to 45°C.

In this 'wet' method, for example a spray dryer can be used to produce dry crosslinked alginate. According to the method reported by Möbus et al. [ref 29], a solution containing Na-ALG with metal salts and ammonia can be spray dried to generate crosslinked M-ALG. Upon drying, ammonia evaporates releasing the metal ions which subsequently crosslink the alginate during the drying stage of the spray drying process.

In this 'wet' method for preparing M-ALG crosslinked particles, if desired, the collected wet M-ALG crosslinked particles may be washed and filtered multiple times, before final drying. The collected wet or dried M-ALG crosslinked particles are preferably washed, preferably with water, in particular deionized water, and filtered at least once, for example 2 to 8 times, such as 7 times.

In another aspect, the present invention provides the use of M-ALG (e.g. Fe-ALG, Zn-ALG) crosslinked particles as an agent for inactivating antibiotics selected from quinolones, tetracyclines, and beta-lactams, preferably for inactivating an antibiotic selected from CIP, LEV, NOR, DOXY, TCN, CEX, AMX, and AMP, preferably CIP.

In a preferred embodiment of this aspect, the M-ALG crosslinked particles used are M-ALG (e.g. Fe-ALG or Zn-ALG) crosslinked particles prepared by the above described 'dry' method, in which the dry M-ALG (e.g. Fe-ALG or Zn-ALG) crosslinked particles have been washed (with water) once.

The Me-ALG crosslinked particles used are M-ALG (e.g. Fe-ALG or Zn-ALG) crosslinked particles prepared by the above described 'wet' method, preferably the 'wet' method in which the dry M-ALG (e.g. Fe-ALG or Zn-ALG) crosslinked particles have been washed (with water) 2 to 8 times (e.g. 7 times).

In another embodiment of this aspect, Fe-ALG crosslinked particles are used, preferably Fe-ALG crosslinked particles prepared by the above described 'dry' method.

## EXAMPLES

### Materials and Methods

#### Materials

Zinc acetate $Zn(CH_3COO)_2$, sodium alginate (Na-ALG), hydroxyethyl piperazineethanesulfonic acid (HEPES), calcium chloride, and nickel(II) chloride were all purchased from Sigma Aldrich. CIP hydrochloride, DOXY hydrochloride, LEV hydrochloride, NOR hydrochloride, AMX, AMP, CEX and aluminium chloride were obtained from TCI Development Co®. Iron chloride ($FeCl_3$) was provided by Merck (Darmstadt, Germany). Sodium chloride (NaCl) and concentrated nitric acid were both bought from Fisher Scientific (UK, Loughborough, England). Copper chloride and TCN hydrochloride were purchased form Acros Chemicals.

Simulated intestinal medium (SIM) was prepared by dissolving 0.238g HEPES and 0.84g NaCl in 100 mL deionised water. The pH was adjusted to 6.5 using diluted HCl or NaOH solutions as required.

#### Preparation of Metal-Alginate powders

Metal-ALG (Fe(III)-ALG, Zn-ALG, Cu-ALG, Ni-ALG, Al-ALG, and Ca-ALG) crosslinked particles were prepared by 'wet' or 'dry' crosslinking methods, and washed, by the following procedures:

- Wet crosslinking (not according with the invention)

ALG solution (3% w/v) was prepared by dissolving 1.5 g Na-ALG in 50 mL water. Obtained solution was then added dropwise to 300 mL $Zn(CH_3COO)_2$ or $FeCl_3$ solutions (10% w/v) using a 10 mL syringe. Formed Zn-ALG or Fe(III)-ALG beads were left to mix using a magnetic stirrer for 30 minutes before being collected and washed one or seven times, one minute each wash, in 300 mL distilled water. Fresh deionised water was used for each washing. On the seventh wash, the deionised water used to wash the Fe(III)-ALG beads was colourless. The washed Zn-ALG and Fe(III)-ALG beads were freeze dried for 48 hours, crushed manually using a pestle and mortar, and stored in labelled vials.

- Dry crosslinking

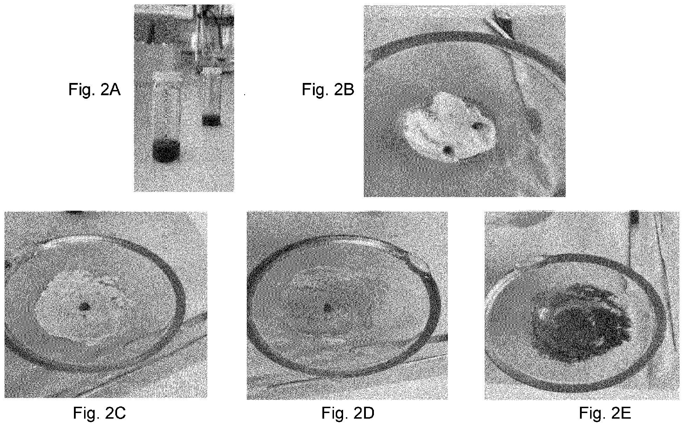

Metal salts (Fe(III), Zn, Cu, Ni, Al, and Ca chloride salts) were each dissolved in a minimal volume of deionised water (about 2 mL) to a concentration equivalent to 2.74mM iron, the solution was added dropwise at a slow rate (0.25 mL per minute) to a mortar containing Na-ALG powder (1g), and mixed manually together using a pestle. The obtained mixture was washed once in 300 mL of deionised water, filtered, and placed in a petri-dish, and left to dry over-night in an oven at 40 °C, before being collected and stored in a labelled glass vial. **Fig. 2A** shows vials containing $FeCl_3$ solution. **Figs. 2B-2E** show, at intervals, progressive dropwise additions of $FeCl_3$ solution being made to initially dry Na-ALG powder **(Fig. 2B),** with mixing, to form moist or near-dry crosslinked alginate particles **(Fig. 2E).**

Metal-ALG (Fe(III)-ALG, Zn-ALG, Cu-ALG, Ni-ALG, Al-ALG, and Ca-ALG) crosslinked particle powders were thus obtained, as identified by the abbreviations indicated in Table 1:

Table 1:

| | |
|---|---|
| Wet-metal-ALG*1w | metal-ALG prepared by wet crosslinking; washed once |
| Wet-metal-ALG*7w | metal-ALG prepared by wet crosslinking; washed 7 times |
| Dry-metal-ALG | metal-ALG prepared by dry crosslinking; washed once |

#### Antibiotics adsorption by metal-ALG crosslinked particles

In order to explore the impact of cations type (i.e. Fe(III), Zn, Cu, Ni, Al, and Ca) on the antibiotics (CIP, LEV, NOR, DOXY, TCN, CEX, AMP, AMX) complexation, 100 mg of Dry-metal-ALG powders were added separately to beakers containing 10 mL antibiotics solution (100 $\mu$g/mL). The final concentration of Dry-metal-ALG powders was 10 mg/mL. The beakers were placed inside a shaking water bath (Grant Scientific, OLS200) operating at 100 rpm and 37.5 °C. Samples (250 µL) were collected at specific time intervals, filtrated, and diluted with water (1 to 8) before being analysed using a UV spectrophotometer. The concentration of remaining antibiotics was calculated using to a standard calibration curve of CIP in water.

Inactivation capacity, adsorption Isotherms, and adsorption kinetic modelling

The inactivation capacity of Wet-Fe-ALG*7w and Dry-Fe-ALG (1mg/mL) was investigated in increased concentrations of CIP in SIM (25, 50, 100, 200, 400, 600, 800, 1000 µg/mL) following the same method mentioned above.

The inactivation capacity (Q) was calculated according to the following equation Eq.1:

$$Q = (Ci - Ceq)/a \dots\dots\dots\dots\dots\dots\dots \text{ (Eq. 1)}$$

where $Ci$ and $Ceq$ are the initial and equilibrium concentrations of CIP (mg/mL) and a is the amount in grams of ALG.

The adsorption isotherm was generated by plotting $Q$ (mg/g) against $Ceq$ for each concentration of CIP. The pseudo-second order model is expressed by the equation as follows:

$$\frac{1}{q_t} = \frac{1}{K_2\, q_{eq}^2} + \frac{1}{q_{eq}} t$$

where $q_t$ and $q_{eq}$ are the amount (mg per g of alginate) of adsorption at equilibrium and at a particular time ($t$), respectively and $K_2$ represents the overall rate constant for pseudo-second order adsorption with the unit of g/mg min. A linear plot will be achieved from the graph of $t/q_t$ versus time.

Atomic Spectrometry

Wet-Fe-ALG*7w and Dry-Fe-ALG powder (30 mg) were weighed accurately in a glass vial containing 5 mL of concentrated nitric acid. The vials were tightly closed, shaken and placed in a drawer over-night to allow digestion of Fe(III)-ALG powder. The amount of released iron was quantified using inductively coupled plasma atomic emission spectrometer (ICP-AES, Ultima 2 C, Jobin Yvon, Horiba).

Antibacterial activity assay

*E.coli* antibacterial activities of CIP solutions (100 µg/mL) before and after inactivation in SIM with Dry-Fe-ALG (1 mg/mL), and Wet-Fe-ALG*7w (1 mg/mL) were determined by the agar well diffusion method. Briefly, *E.coli* inoculum (0.5 McFarland) was spread, using sterile cotton swabs dipped into the bacterial suspension, on nutrient agar plates [ref 20]. Subsequently, wells (8 mm) were cut out of the *E.coli* inoculated agar plates using sterilised cork borer. Wells were filled with 50 µL (equivalent to 5 µg CIP before inactivation) CIP solutions before and after the inactivation with that above mentioned powders. All solutions were sterilised by filtration (0.45 Micron, Millipore, Millex). The plates were then incubated at 37 °C for 24 h and the zones of growth inhibition were measured in mm.

**Results and Discussion**

Dry crosslinking: impact of cation type on antibiotics inactivation

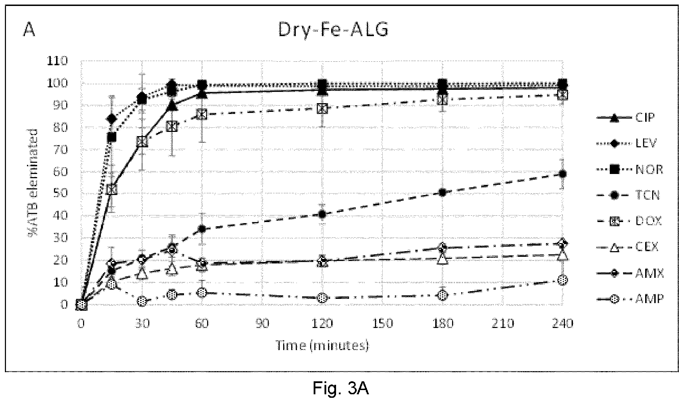

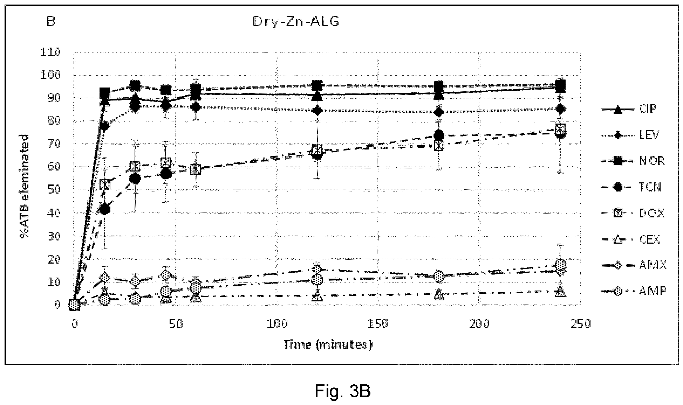

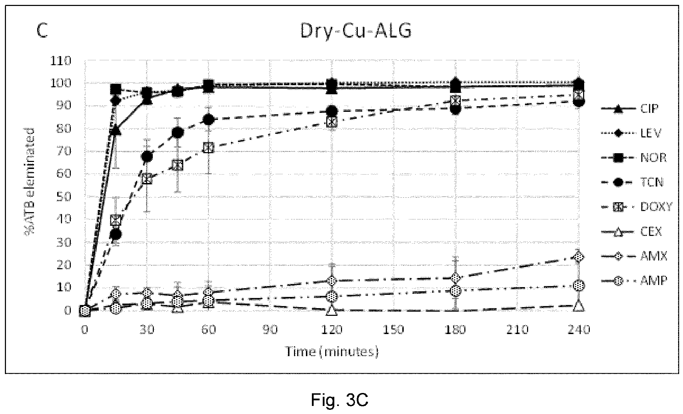

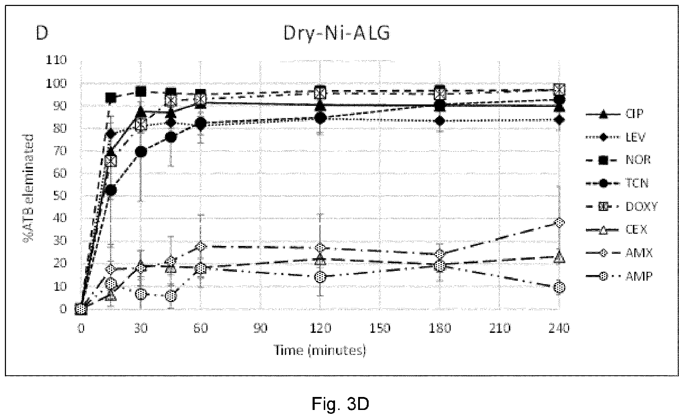

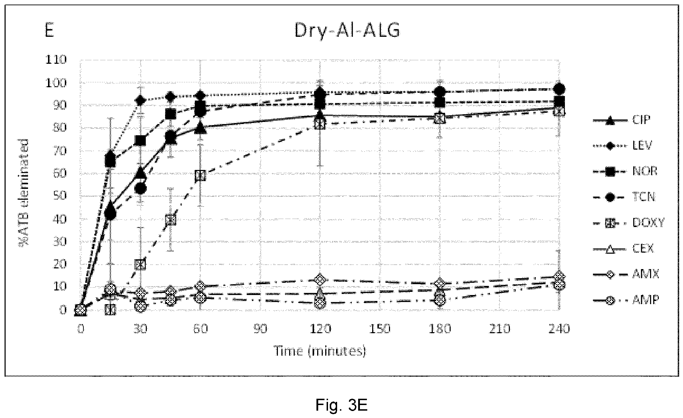

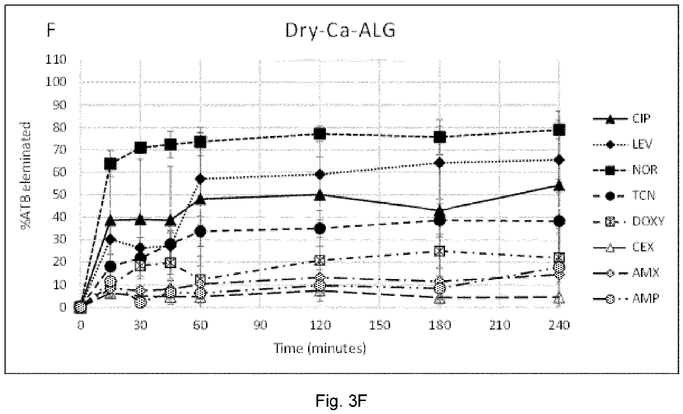

Initially, the ability of different Dry-metal-ALG crosslinked particles to remove antibiotics was established. **Figs. 3A-3F** represent the inactivation percentage (%) of CIP, LEV, NOR, TCN, DOXY, CEX, AMP, and AMX solutions (100 µg/mL) in water using 10 mg/mL of Dry-Fe-ALG, Dry-Zn-ALG, Dry-Cu-ALG, Dry-Ni-ALG, Dry-Al-ALG, and Dry-Ca-ALG, respectively. Apart from Ca-ALG, all metal-ALG crosslinked particles prepared by the dry method showed significant capacity at inactivating both quinolones (CIP, LEV, and NOR) and tetracyclines (TCN and DOXY). Inactivation capacity and inactivation kinetic towards both antibiotics groups were rather high and fast, respectively. For instance, Dry-Zn-ALG and Dry-Cu-ALG particles were able to inactivate more than 80% of CIP and TCN in the first 15 minutes and more than 90% was removed in the first hour. Dry-Fe-ALG showed an excellent inactivation capacity towards CIP. However, it was less effective against TCN with a maximum of 60% elimination reached at approximately 3 hours. Ni-ALG and Al-ALG powder exhibited excellent inactivation against TCN with the maximum inactivation reached in 180 minutes. However, they were slightly less effective toward CIP with almost 80% removed in 120 minutes. On the other hand, Ca-ALG particles showed weaker and poorly reproducible inactivation towards all tested antibiotics.

The inactivation capacity of all Dry-metal-ALG particles was noticeably less effective towards beta-lactams (CEX, AMP, AMX) with approximately only 20% maximum inactivation even after 4 hours.

Wet crosslinking: impact of washing number on antibiotics inactivation (not according with the invention)

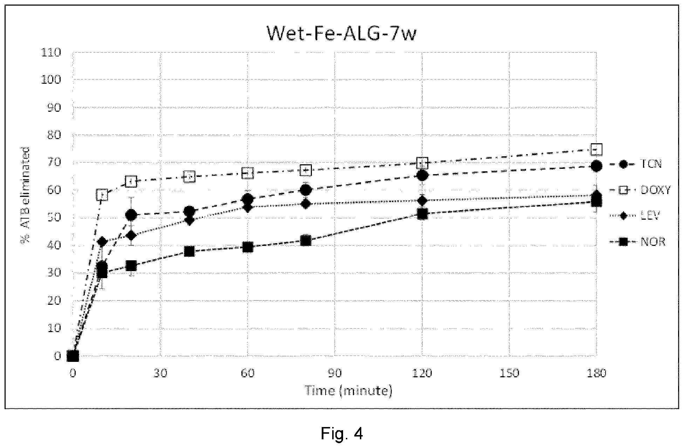

**Fig. 4** shows the percentage of elimination of TCN, DOXY, LEV, and NOR (100 $\mu$g/mL) in water using 10 mg/mL Wet-Fe-ALG*7w. Wet-crosslinked particles were less effective in inactivating the quinolones LEV and NOR (up to 60% eliminated in 3 hours **(Fig. 4))** and the tetracycline DOXY (up to 70%) than the corresponding salt obtained by the dry crosslinking method (>90 % eliminated in the first 15-30 minutes **(Fig. 3A)).** On the contrary, Wet-Fe-ALG*7w particles were slightly more efficacious at eliminating the tetracycline TCN from water as compared with Dry-Fe-ALG **(Fig. 4** and **Fig. 3A).**

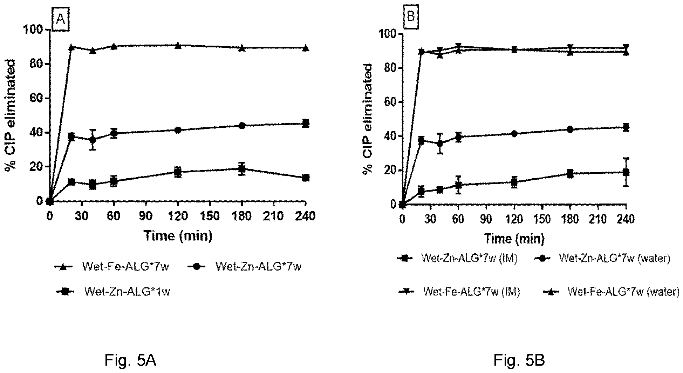

**Fig. 5A** represents the inactivation percentage of CIP solution (100 $\mu$g/mL) in water using 10 mg/mL Wet-Zn-ALG*1w, Wet-Zn-ALG*7w, and Wet-Fe-ALG*7w. Wet-Fe-ALG*7w inactivated up to 90% of CIP in less than 20 min. However, it was not possible to measure the absorbance of CIP when Wet-Fe-ALG*1w powder was used as the colour of the inactivation medium changed immediately, resulting in very high absorbance readings (data not shown). On the other hand, Wet-Zn-ALG*7w, and Wet-Zn-ALG*1w powders inactivated 45.3% and 13.6% of CIP respectively. In addition to cross-linked $Zn^{2+}$ and $Fe^{3+}$, Zn-ALG and Fe(III)-ALG powder entrapped free cations that are known to be washable with water. It was reported that 7 washes were needed to wash out all the free calcium ($Ca^{2+}$) from Ca-ALG and Ca-pectin beads [refs 5,18]. Therefore, if powders are not washed enough (i.e. Wet-Zn-ALG*1w and Wet-Fe-ALG*1w), the free $Zn^{2+}$ and $Fe^{3+}$ could be released into the inactivation medium and complex CIP in solution rather than on crosslinking sites. The external complexation does not allow the immobilisation of CIP on the ALG powder and would therefore negatively affect the overall inactivation efficiency of the Zn-ALG or Fe(III)-ALG powders.

Impact of inactivation medium on CIP inactivation

The impact of inactivation medium (water vs SIM) on the inactivation capacity of Zn-ALG and Fe(III)-ALG was investigated. **Fig. 5B** represents the inactivation percentage of CIP (100 $\mu$g/mL) in water and SIM using 10mg/mL Wet-Fe-ALG*7w or Wet-Zn-ALG*7w.

In contrast with the Zn-ALG powder that inactivated significantly less CIP in SIM (about 18%) than in water (about 40%), Fe(III)-ALG powder revealed an identical high inactivation capacity in both mediums (> 90%). The lower inactivation capacity of Zn-ALG in SIM may be explained by the ion exchange process, whereby $Zn^{2+}$ exchanges with $Na^+$ present in the SIM [ref 18]. This exchange of metal ions leads to the release of $Zn^{2+}$ into the SIM, and thus to external complexation. This does not seem to be the case with the Fe(III)-ALG system where both mediums show high inactivation capacity, suggesting a more resistant Fe(III)-ALG crosslinking.

Inactivation capacity, inactivation isotherms, and adsorption kinetic modelling

Wet crosslinking of ALG with metals is an established field, where ALG is usually dissolved in water and added dropwise to a cross-linker solution [refs 13, 16]. The diffusion of the cations (i.e. cross-linkers) into the ALG matrix allows a deep crosslinking of ALG. To the authors' knowledge, the present study is the first to report the possible application of dry crosslinking (i.e. ALG used as dry alginate powder rather than alginate in solution) to generate a superficial crosslinking. In addition to rendering the complexation sites more accessible to antibiotics' molecules due to their superficial positions, dry crosslinking is a one-step method that does not necessitate ALG dissolving, several washings, nor long hours of freeze drying. The concentration of the $Fe^{3+}$ solution used for the dry crosslinking of ALG in this study was calculated in such a way that the final concentration of $Fe^{3+}$ per gram ALG equals that of wet cross-linked powder after 7 washes and before the inactivation experiment (data not shown). However, it is worth mentioning that obtained dry crosslinked ALG powder underwent one washing in order to eliminate any free $Fe^{3+}$ and thus prevent possible in-solution external complexation of CIP.

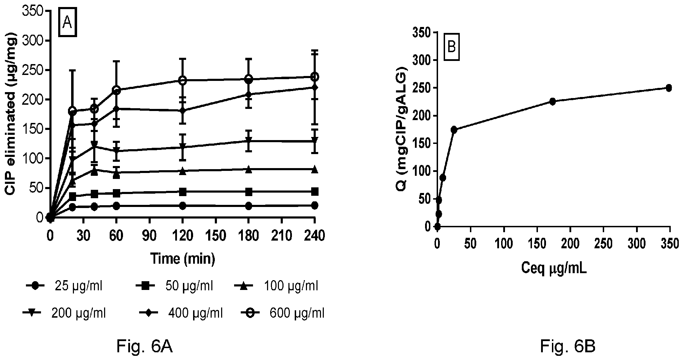

Wet-Fe-ALG*7w powder (1mg/mL) was used to establish the inactivation capacity and isotherm of wet cross-linked particles in SIM containing CIP at concentrations ranging from 25 to 600 $\mu$g/mL **(Fig. 6A).** Identical inactivation profiles, with consistently higher inactivation equilibriums reached at approximately 20 minutes, were observed as the initial CIP concentration increased. The maximum ALG inactivation capacity, calculated according to Eq.1, was about 280 mg CIP per gram ALG for an initial CIP solution concentration of 600 mg/mL. The concentration of CIP at equilibrium were plotted against the ALG inactivation capacities in order to obtain the adsorption isotherm for Wet-Fe-ALG*7w (**Fig. 6B**). A type I isotherm, concave towards the x-axis, was generated [ref 23], suggesting a limited number of complexing sites, hence more than one CIP molecule complexed by one complexation site (i.e. $Fe^{3+}$ site). This was in a good agreement with the expected bidentate ligand behaviour of CIP towards Fe complexation [ref 15]. To investigate this hypothesis further, atomic absorption spectrometry was used to quantify the amount of $Fe^{3+}$ retained by ALG after the inactivation experiment.

Table 2 shows mMoles of CIP and $Fe^{3+}$ retained by one gram ALG and their final molar ratio after the inactivation experiment. As shown in Table 2, 1.614 mMole $Fe^{3+}$ were found per one gram ALG, resulting in a CIP : Fe molar ratio of 0.46. This means that virtually each two available $Fe^{3+}$ sites were able to complex one CIP molecule (Table 2). This assumption is unlikely to happen as at least one CIP molecule, and most likely two, are required per each complexation site. It seems therefore plausible to conclude that not all the $Fe^{3+}$ sites were accessible/available for CIP molecules, namely if considering the deep crosslinking that a wet crosslinking process would produce.

Table 2:

|  | mMole $Fe^{3+}$ per g ALG | mMole CIP per g ALG | Molar ratio CIP : Fe |
|---|---|---|---|
| Wet crosslinking | 1.614 | 0.754 | 0.46 |
| Dry crosslinking | 0.397 | 0.829 | 2.08 |

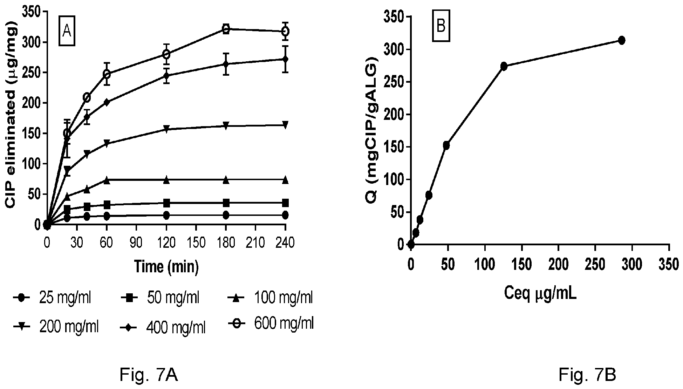

The inactivation capacity of Dry-Fe-ALG (1mg/mL) towards increased concentrations of CIP (25 to 600 µg/mL) in SIM are shown in (Fig. 7A). The inactivation profiles generated using dry cross-linked ALG were relatively comparable to those of the wet cross-linked ALG (Fig. 6A). However, the maximum inactivation capacity of dry cross-linked ALG was significantly higher (320 mg (in dry method) vs 280 mg (wet method) CIP per gram ALG), indicating a higher accessibility of CIP to the superficial complexation sites. A similar type I adsorption isotherm for the Dry-Fe-ALG was obtained (Fig. 7B). Interestingly, by quantifying retained $Fe^{3+}$ (mMoles) per gram ALG (Table 2), the bidentate nature of the obtained complex was confirmed (i.e. CIP : Fe ratio (2:1)) that could suggest a complete saturation of all $Fe^{3+}$ sites within the Fe(III)-ALG matrix, reflecting their better accessibility for CIP molecules.

Table 3 summarises the maximum inactivation capacity (mg antibiotic per 1g Dry-Fe-ALG) of different antibiotics using 1mg/mL Dry-Fe-ALG and an initial concentration of antibiotics of 1000 µg/mL.

Table 3:

| Antibiotic | CIP | NOR | LEV | TCN | DOXY |
|---|---|---|---|---|---|
| mg ATB/g ALG | 374 | 372 | 498 | 128 | 154 |

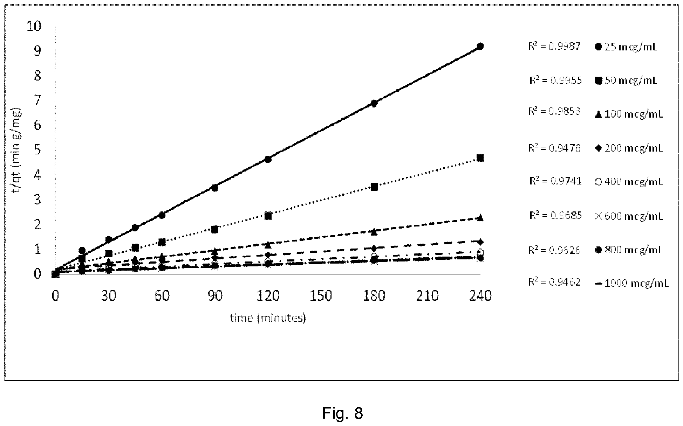

Fig. 8 shows a plot of the linearized form of the pseudo-second order for the adsorption of CIP (from initial concentrations ranging from 25 to 1000 mcg/mL) onto Dry-Fe-ALG particles (1mg/mL). The correlation coefficients for the linear plots of t/qt against time are all greater than 0.94, suggesting the chemisorption to be the main mechanism of adsorption, which in good agreement with the discussed chemical complexation of CIP molecules with iron sites.

Zones of inhibition of *E.coli*

In this part of the study, CIP solutions at a concentration equivalent to its active faecal concentration (i.e. 100 µg/mL) [ref 26] were inactivated using Dry-Fe-ALG (1 mg/mL), Wet-Fe-ALG*w7 (1 mg/mL) or Wet-Zn-ALG*7w (10 mg/mL). The microbiological activity of CIP solutions before and after inactivation was subsequently investigated by measuring the zone of inhibition of *E.coli* as a model of microbiota bacterial species.

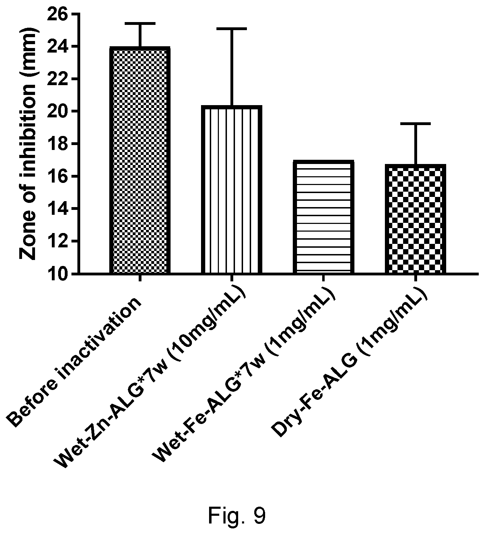

Fig. 9 shows the zones of inhibition (mm) obtained on *E.coli* plates after the addition of 50 µL of CIP solutions (100 mg/mL) before and after inactivation (= 5µg CIP before inactivation). The zones of inhibition were significantly reduced from 22 mm using CIP solution before inactivation to the range of 16-17 mm when treated with a similar volume of CIP solutions inactivated with 1 mg/mL Wet-Fe-ALG*7w or Dry-Fe-ALG powder (P < 0.05). However, no significant decrease in the diameter of the zone of inhibition was observed when *E.coli* plates were treated with 50 µL CIP solution after inactivation with ten times higher concentration (10 mg/mL) Wet-Zn-ALG*7w.

The zone of inhibition results clearly highlight a significant reduction of CIP antimicrobial activity using a clinically conceivable concentration (1 mg/mL) of Fe(III)-ALG powder. If a similar inactivation capacity is reproduced *in vivo,* an estimated dose of <1 g Fe(lll)-ALG daily would be needed considering the limited volume of colonic fluids where the inactivation is supposed to happen [ref 27].

**References**

1. Resistance, R.o.A., Tackling drug-resistant infections globally: final report and recommendations. 2016: Review on antimicrobial resistance.

2. National Academies of Sciences, E. and Medicine, Environmental chemicals, the human microbiome, and health risk: A research strategy. 2018: National Academies Press.

3. Khoder, M., N. Tsapis, and E. Fattal, Mechanisms of antibiotic resistance and delivery strategies to prevent its emergence. Journal of Drug Delivery Science and Technology, 2010. 20(6): p. 407-418.

4. Khoder, M., et al., Removal of residual colonic ciprofloxacin in the rat by activated charcoal entrapped within zinc-pectinate beads. European Journal of Pharmaceutical Sciences, 2010. 41(2): p. 281-288.

5. Khoder, M., et al., Removal of ciprofloxacin in simulated digestive media by activated charcoal entrapped within zinc-pectinate beads. International Journal of Pharmaceutics, 2009. 379(2): p. 251-259.

6. Bourgeois, S., et al., Colonic delivery of β-lactamases does not affect amoxicillin pharmacokinetics in rats. Journal of pharmaceutical sciences, 2008. 97(5): p. 1853-1863.

7. Kaleko, M., et al., Development of SYN-004, an oral beta-lactamase treatment to protect the gut microbiome from antibiotic-mediated damage and prevent Clostridium difficile infection. Anaerobe, 2016. 41: p. 58-67.

8. Reynaud, F., et al., Spray-dried chitosan-metal microparticles for ciprofloxacin adsorption: Kinetic and equilibrium studies. Soft Matter, 2011. 7(16): p. 7304-7312.

9. de Gunzburg, J., et al., Targeted adsorption of molecules in the colon with the novel adsorbent-based Medicinal Product, DAV132: A proof of concept study in healthy subjects. The Journal of Clinical Pharmacology, 2015. 55(1): p. 10-16.

10. de Gunzburg, J., et al., Protection of the Human Gut Microbiome From Antibiotics. The Journal of Infectious Diseases, 2017. 217(4): p. 628-636.

11. Drusano, G., et al., Absolute oral bioavailability of ciprofloxacin. Antimicrobial agents and chemotherapy, 1986. 30(3): p. 444-446.

12. Ramon, J., et al., Transepithelial intestinal excretion of ciprofloxacin in humans. Clinical infectious diseases, 2001. 32(5): p. 822-823.

13. Dubinsky, V., et al., Prolonged Antibiotic Treatment Generates a Fluoroquinolone Resistant Gut Microbiome and Collateral Multi-Drug Resistance. bioRxiv, 2018: p. 467001.

14. Haak, B.W., et al., Long-term impact of oral vancomycin, ciprofloxacin and metronidazole on the gut microbiota in healthy humans. Journal of Antimicrobial Chemotherapy, 2018. 74(3): p. 782-786.

15. Serafin, A. and A. Stańczak, The complexes of metal ions with fluoroquinolones. Russian Journal of Coordination Chemistry, 2009. 35(2): p. 81-95.

16. Uivarosi, V., Metal complexes of quinolone antibiotics and their applications: an update. Molecules, 2013. 18(9): p. 11153-11197.

17. Khoder, M., et al., Potential use of the Maillard reaction for pharmaceutical applications: gastric and intestinal controlled release alginate-albumin beads. Pharmaceutics, 2019. 11(2): p. 83.

18. Dalaty, A.A., et al., Effect of non-cross-linked calcium on characteristics, swelling behaviour, drug release and mucoadhesiveness of calcium alginate beads. Carbohydrate Polymers, 2016. 140: p. 163-170.

19. Zhang, X., et al., Novel alginate particles decorated with nickel for enhancing ciprofloxacin removal: Characterization and mechanism analysis. Ecotoxicology and Environmental Safety, 2019. 169: p. 392-401.

20. Lalitha, M., Manual on antimicrobial susceptibility testing. Performance standards for antimicrobial testing: Twelfth Informational Supplement, 2004. 56238: p. 454-456.

21. Obaleye, J., et al., Toxicological studies and antimicrobial properties of some Iron (III) complexes of Ciprofloxacin. African Journal of Biotechnology, 2007. 6(24).

22. Zaid, A.A., et al., Effect of ionic strength on the stabilities of ciprofloxacin - Metal complexes. Journal of Saudi Chemical Society, 2013. 17(1): p. 43-45.

23. Rouquerol, J., et al., Adsorption by powders and porous solids: principles, methodology and applications. 2013: Academic press.

24. Brumfitt, W., et al., Changes in the pharmacokinetics of ciprofloxacin and fecal flora during administration of a 7-day course to human volunteers. Antimicrobial Agents and Chemotherapy, 1984. 26(5): p. 757-761.

25. Pecquet, S., S. Ravoire, and A. Andremont, Faecal excretion of ciprofloxacin after a single oral dose and its effect on faecal bacteria in healthy volunteers. Journal of Antimicrobial Chemotherapy, 1990. 26(1): p. 125-129.

26. Edlund, C., L. Lindqvist, and C.E. Nord, Norfloxacin binds to human fecal material. Antimicrobial agents and chemotherapy, 1988. 32(12): p. 1869-1874.

27. Schiller, C., et al., Intestinal fluid volumes and transit of dosage forms as assessed by magnetic resonance imaging. Alimentary pharmacology & therapeutics, 2005. 22(10): p. 971-979.

28. Carlotti, B., et al., Complexes of tetracyclines with divalent metal cations investigated by stationary and femtosecond-pulsed techniques, 2011, Phys. Chem. Chem. Phys., 2012, 14, 823-834

29. Möbus K, Siepmann J, Bodmeier R., Zinc-alginate microparticles for controlled pulmonary delivery of proteins prepared by spray-drying. European Journal of Pharmaceutics and Biopharmaceutics. 2012 May 1;81(1):121-30

**Claims**

1. A method for producing metal-alginate crosslinked particles wherein the metal is an ion of iron, zinc, copper, nickel, aluminium, or calcium, comprising:

   providing sodium-alginate powder;
   providing a solution of a metal salt wherein the metal salt is a salt of iron, zinc, copper, nickel, aluminium, or calcium;
   adding the metal salt solution, gradually or portionwise, to the sodium-alginate powder and mixing, to form metal-alginate crosslinked particles in a moist or near-dry state;
   drying, or leaving to dry, the formed moist or near-dry metal-alginate crosslinked particles to form dry metal-alginate crosslinked particles;
   optionally washing the dry metal-alginate crosslinked particles, followed by filtering and drying.

2. The method according to claim 1, wherein the dry metal-alginate crosslinked particles are washed with water and filtered, at least once.

3. The method according to claim 1 or claim 2, wherein the method is for producing metal-alginate crosslinked particles wherein the metal is an ion of iron, zinc, copper, nickel, or aluminium, and the metal salt added is a salt of iron, zinc, copper, nickel, or aluminium.

4. The method according to any one of claims 1 to 3, wherein a fluidised bed dryer is used to form the dry metal-alginate crosslinked particles.

5. The method according to any one of claims 1 to 4, wherein the method is for producing metal-alginate crosslinked particles wherein the metal is an ion of iron or zinc, and the metal salt added is a salt of iron or zinc.

6. The method according to any one of claims 1 to 5, wherein the method is for producing Fe(III)-alginate crosslinked particles, and the solution added is an aqueous iron (III) chloride solution.

7. Metal-alginate crosslinked particles prepared by the method of any one of claims 1 to 5.

8. Metal-alginate crosslinked particles according to claim 7, wherein the metal-alginate crosslinked particles are Fe(III)-alginate crosslinked particles prepared by the method of claim 6.

9. Use of metal-alginate crosslinked particles as defined in claim 7 or claim 8, as an agent for inactivating antibiotics selected from quinolones, tetracyclines, and beta-lactams.

10. The use according to claim 9 for inactivating an antibiotic selected from ciprofloxacin, levofloxacin, norfloxacin, doxycycline, tetracycline, amoxicillin, ampicillin, and cephalexin, preferably ciprofloxacin.

11. The use according to claim 9 or claim 10, wherein the metal-alginate crosslinked particles have been washed with water at least once.

**Patentansprüche**

1. Verfahren zum Erzeugen von Metallalginat-vernetzten Teilchen, wobei es sich bei dem Metall um ein Ion von Eisen, Zink, Kupfer, Nickel, Aluminium oder Calcium handelt, das Folgendes umfasst:

   Bereitstellen von Natriumalginatpulver;
   Bereitstellen einer Lösung eines Metallsalzes, wobei es sich bei dem Metallsalz um ein Salz von Eisen, Zink, Kupfer, Nickel, Aluminium oder Calcium handelt;
   Zugeben der Metallsalzlösung nach und nach oder portionsweise zu dem Natriumalginatpulver und Mischen, um Metallalginat-vernetzte Teilchen in einem feuchten oder nahezu trockenen Zustand zu bilden;
   Trocknen oder Trocknenlassen der gebildeten feuchten oder fast trockenen Metallalginat-vernetzten Teilchen, um trockene Metallalginat-vernetzte Teilchen zu erhalten;
   optional Waschen der trockenen, Metallalginat-vernetzten Teilchen gefolgt von Filtern und Trocknen.

**2.** Verfahren nach Anspruch 1, wobei die trockenen, Metallalginat-vernetzten Teilchen mindestens einmal mit Wasser gewaschen und filtriert werden.

**3.** Verfahren nach Anspruch 1 oder Anspruch 2, wobei das Verfahren zum Produzieren von Metallalginat-vernetzten Teilchen dient, wobei es sich bei dem Metall um ein Ion von Eisen, Zink, Kupfer, Nickel oder Aluminium handelt und das zugegebene Metallsalz ein Salz von Eisen, Zink, Kupfer, Nickel oder Aluminium ist.

**4.** Verfahren nach einem der Ansprüche 1 bis 3, wobei ein Wirbelschichttrockner zum Bilden der trockenen, Metallalginat-vernetzten Teilchen verwendet wird.

**5.** Verfahren nach einem der Ansprüche 1 bis 4, wobei das Verfahren zum Produzieren von Metallalginat-vernetzten Teilchen dient, wobei es sich bei dem Metall um ein Eisen- oder Zinkion handelt und das zugegebene Metallsalz ein Eisen- oder Zinksalz ist.

**6.** Verfahren nach einem der Ansprüche 1 bis 5, wobei das Verfahren zum Produzieren von Fe(III)-Alginat-vernetzten Teilchen dient und die zugegebene Lösung eine wässrige Eisen(III)-chlorid-Lösung ist.

**7.** Metallalginat-vernetzte Teilchen, hergestellt gemäß dem Verfahren nach einem der Ansprüche 1 bis 5.

**8.** Metallalginat-vernetzte Teilchen nach Anspruch 7, wobei es sich bei den Metallalginat-vernetzten Teilchen um Fe(III)-Alginat-vernetzte Teilchen handelt, die gemäß dem Verfahren des Anspruchs 6 hergestellt sind.

**9.** Verwendung von Metallalginat-vernetzten Teilchen nach Anspruch 7 oder Anspruch 8 als Mittel zum Inaktivieren von Antibiotika, ausgewählt aus Chinolonen, Tetracyclinen und Beta-Lactamen.

**10.** Verwendung nach Anspruch 9 zum Inaktivieren eines Antibiotikums, ausgewählt aus Ciprofloxacin, levofloxacin, Norfloxacin, Doxycyclin, Tetracyclin, Amoxicillin, Ampicillin und Cephalexin, bevorzugt Ciprofloxacin.

**11.** Verwendung nach Anspruch 9 oder Anspruch 10, wobei die Metallalginat-vernetzten Teilchen mindestens einmal mit Wasser gewaschen wurden.

**Revendications**

**1.** Procédé de production de particules réticulées d'alginate métallique, dans lequel le métal est un ion de fer, de zinc, de cuivre, de nickel, d'aluminium ou de calcium, comprenant :

la fourniture de poudre d'alginate de sodium ;
la fourniture d'une solution d'un sel métallique dans lequel le sel métallique est un sel de fer, de zinc, de cuivre, de nickel, d'aluminium ou de calcium ;
l'ajout progressif ou par portions de la solution de sel métallique à la poudre d'alginate de sodium et le mélange pour former des particules réticulées d'alginate métallique à l'état humide ou presque sec ;
le séchage, ou la mise à sécher, des particules réticulées d'alginate métallique humides ou presque sèches formées pour former des particules réticulées d'alginate métallique sèches ;
le lavage facultatif des particules réticulées d'alginate métallique sèches, suivi d'une filtration et d'un séchage.

**2.** Procédé selon la revendication 1, dans lequel les particules réticulées d'alginate métallique sèches sont lavées à l'eau et filtrées, au moins une fois.

**3.** Procédé selon la revendication 1 ou la revendication 2, dans lequel le procédé est destiné à produire des particules réticulées d'alginate métallique, dans lequel le métal est un ion de fer, de zinc, de cuivre, de nickel ou d'aluminium, et le sel métallique ajouté est un sel de fer, de zinc, de cuivre, de nickel ou d'aluminium.

**4.** Procédé selon l'une quelconque des revendications 1 à 3, dans lequel un séchoir à lit fluidisé est utilisé pour former les particules réticulées d'alginate métallique sèches.

**5.** Procédé selon l'une quelconque des revendications 1 à 4, dans lequel le procédé est destiné à produire des particules réticulées d'alginate métallique, dans lequel le métal est un ion de fer ou de zinc, et le sel métallique ajouté est un sel de fer ou de zinc.

6. Procédé selon l'une quelconque des revendications 1 à 5, dans lequel le procédé est destiné à produire des particules réticulées d'alginate de Fe(III), et la solution ajoutée est une solution aqueuse de chlorure de fer (III).

7. Particules réticulées d'alginate métallique préparées par le procédé selon l'une quelconque des revendications 1 à 5.

8. Particules réticulées d'alginate métallique selon la revendication 7, dans lesquelles les particules réticulées d'alginate métallique sont des particules réticulées d'alginate de Fe(III) préparées par le procédé selon la revendication 6.

9. Utilisation de particules réticulées d'alginate métallique telles que définies dans la revendication 7 ou la revendication 8, comme agent d'inactivation d'antibiotiques choisi parmi les quinolones, les tétracyclines et les bêta-lactames.

10. Utilisation selon la revendication 9 pour inactiver un antibiotique choisi parmi la ciprofloxacine, la lévofloxacine, la norfloxacine, la doxycycline, la tétracycline, l'amoxicilline, l'ampicilline et la céphalexine, de préférence la ciprofloxacine.

11. Utilisation selon la revendication 9 ou la revendication 10, dans laquelle les particules réticulées d'alginate métallique ont été lavées à l'eau au moins une fois.

Fig. 1A

Fig. 1B

Fig. 2A

Fig. 2B

Fig. 2C

Fig. 2D

Fig. 2E

Fig. 3A

Fig. 3B

Fig. 3C

Fig. 3D

Fig. 3E

Fig. 3F

Fig. 4

Fig. 5A          Fig. 5B

Fig. 6A

Fig. 6B

Fig. 7A

Fig. 7B

Fig. 8

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007205157 A1

- CN 108339526 B

**Non-patent literature cited in the description**

- **ZHANG et al.** *Ecotoxological and Environmental Safety*, 2019, vol. 169, 392-401
- **SINGH et al.** *Journal of Materials Research and Technology*, 2014, vol. 3 (3), 195-202
- **KONWAR et al.** *RSC Advances*, 2015, vol. 5, 81573-81582
- **CUERVO LAMBAQUE, E et al.** *Environmental Science and Pollution Research International*, vol. 26 (7), 6532-6544
- **RESISTANCE, R.O.A.** Tackling drug-resistant infections globally: final report and recommendations.. *Review on antimicrobial resistance*, 2016
- National Academies of Sciences, E. and Medicine. Environmental chemicals, the human microbiome, and health risk: A research strategy. National Academies Press, 2018
- **KHODER, M** ; **N. TSAPIS** ; **E. FATTAL**. Mechanisms of antibiotic resistance and delivery strategies to prevent its emergence. *Journal of Drug Delivery Science and Technology*, 2010, vol. 20 (6), 407-418
- **KHODER, M. et al.** Removal of residual colonic ciprofloxacin in the rat by activated charcoal entrapped within zinc-pectinate beads. *European Journal of Pharmaceutical Sciences*, 2010, vol. 41 (2), 281-288
- **KHODER, M. et al.** Removal of ciprofloxacin in simulated digestive media by activated charcoal entrapped within zinc-pectinate beads. *International Journal of Pharmaceutics*, 2009, vol. 379 (2), 251-259
- **BOURGEOIS, S et al.** Colonic delivery of β-lactamases does not affect amoxicillin pharmacokinetics in rats. *Journal of pharmaceutical sciences*, 2008, vol. 97 (5), 1853-1863
- **KALEKO, M et al.** Development of SYN-004, an oral beta-lactamase treatment to protect the gut microbiome from antibiotic-mediated damage and prevent Clostridium difficile infection.. *Anaerobe*, 2016, vol. 41, 58-67
- **REYNAUD, F. et al.** Spray-dried chitosan-metal microparticles for ciprofloxacin adsorption: Kinetic and equilibrium studies. *Soft Matter*, 2011, vol. 7 (16), 7304-7312

- **DE GUNZBURG, J. et al.** Targeted adsorption of molecules in the colon with the novel adsorbent-based Medicinal Product, DAV132: A proof of concept study in healthy subjects. *The Journal of Clinical Pharmacology*, 2015, vol. 55 (1), 10-16
- **DE GUNZBURG, J. et al.** Protection of the Human Gut Microbiome From Antibiotics. *The Journal of Infectious Diseases*, 2017, vol. 217 (4), 628-636
- **DRUSANO, G. et al.** Absolute oral bioavailability of ciprofloxacin. *Antimicrobial agents and chemotherapy*, 1986, vol. 30 (3), 444-446
- **RAMON, J. et al.** Transepithelial intestinal excretion of ciprofloxacin in humans. *Clinical infectious diseases*, 2001, vol. 32 (5), 822-823
- **DUBINSKY, V. et al.** Prolonged Antibiotic Treatment Generates a Fluoroquinolone Resistant Gut Microbiome and Collateral Multi-Drug Resistance. *bioRxiv*, 2018, 467001
- **HAAK, B.W. et al.** Long-term impact of oral vancomycin, ciprofloxacin and metronidazole on the gut microbiota in healthy humans. *Journal of Antimicrobial Chemotherapy*, 2018, vol. 74 (3), 782-786
- **SERAFIN, A** ; **A. STAŃCZAK**. The complexes of metal ions with fluoroquinolones. *Russian Journal of Coordination Chemistry*, 2009, vol. 35 (2), 81-95
- **UIVAROSI, V.** Metal complexes of quinolone antibiotics and their applications: an update. *Molecules*, 2013, vol. 18 (9), 11153-11197
- **KHODER, M. et al.** Potential use of the Maillard reaction for pharmaceutical applications: gastric and intestinal controlled release alginate-albumin beads. *Pharmaceutics*, 2019, vol. 11 (2), 83
- **DALATY, A.A. et al.** Effect of non-cross-linked calcium on characteristics, swelling behaviour, drug release and mucoadhesiveness of calcium alginate beads. *Carbohydrate Polymers*, 2016, vol. 140, 163-170

- **ZHANG, X. et al.** Novel alginate particles decorated with nickel for enhancing ciprofloxacin removal: Characterization and mechanism analysis. *Ecotoxicology and Environmental Safety*, 2019, vol. 169, 392-401
- **LALITHA, M.** Performance standards for antimicrobial testing: Twelfth Informational Supplement. *Manual on antimicrobial susceptibility testing.*, 2004, vol. 56238, 454-456
- **OBALEYE, J. et al.** Toxicological studies and antimicrobial properties of some Iron (III) complexes of Ciprofloxacin. *African Journal of Biotechnology*, 2007, vol. 6 (24)
- **ZAID, A.A et al.** Effect of ionic strength on the stabilities of ciprofloxacin - Metal complexes. *Journal of Saudi Chemical Society*, 2013, vol. 17 (1), 43-45
- **ROUQUEROL, J. et al.** Adsorption by powders and porous solids: principles, methodology and applications. Academic press, 2013
- **BRUMFITT, W. et al.** Changes in the pharmacokinetics of ciprofloxacin and fecal flora during administration of a 7-day course to human volunteers. *Antimicrobial Agents and Chemotherapy*, 1984, vol. 26 (5), 757-761

- **PECQUET, S.** ; **S. RAVOIRE** ; **A. ANDREMONT**. Faecal excretion of ciprofloxacin after a single oral dose and its effect on faecal bacteria in healthy volunteers. *Journal of Antimicrobial Chemotherapy*, 1990, vol. 26 (1), 125-129
- **EDLUND, C.** ; **L. LINDQVIST** ; **C.E. NORD**. Norfloxacin binds to human fecal material.. *Antimicrobial agents and chemotherapy*, 1988, vol. 32 (12), 1869-1874
- **SCHILLER, C. et al.** Intestinal fluid volumes and transit of dosage forms as assessed by magnetic resonance imaging. *Alimentary pharmacology & therapeutics*, 2005, vol. 22 (10), 971-979
- **CARLOTTI, B. et al.** Complexes of tetracyclines with divalent metal cations investigated by stationary and femtosecond-pulsed techniques. *Phys. Chem. Chem. Phys.*, 2011, vol. 14, 823-834
- **MÖBUS K** ; **SIEPMANN J** ; **BODMEIER R.** Zinc-alginate microparticles for controlled pulmonary delivery of proteins prepared by spray-drying.. *European Journal of Pharmaceutics and Biopharmaceutics*, 01 May 2012, vol. 81 (1), 121-30